# DEMANDE DE BREVET EUROPEEN

(11) **EP 1 559 409 A1**
(43) Date de publication de la demande: **03.08.2005**
(21) Numéro de dépôt: 05290111.3
(22) Date de dépôt: 19.01.2005
(51) Int. Cl.: A61K 7/13

(54) **Composition de teinture des fibres kératiniques contenant une alcool oxydase et une silicone aminée ou oxyalkylénée, procédé mettant en oeuvre cette composition**

(30) Priorité: 28.01.2004 FR 0400782
(71) Demandeur: L'OREAL, 75008 Paris (FR)
(72) Inventeur: Plos, Grégory, Tokyo 158-0097 (JP)
(74) Mandataire: Casalonga, Axel

(57) **Abrégé**

La présente demande concerne une composition pour la teinture des fibres kératiniques, et en particulier les fibres kératiniques humaines telles que les cheveux, contenant, dans un milieu approprié pour la teinture, au moins un précurseur de colorant d'oxydation, au moins une enzyme alcool oxydase, au moins un substrat pour ladite enzyme et au moins une silicone aminée ou oxyalkylénée. Cette demande concerne encore un procédé de teinture des fibres kératiniques qui consiste à appliquer cette composition ainsi qu'un "kit" de teinture.

## Description

La présente invention a pour objet une composition pour la teinture des fibres kératiniques, en particulier des fibres kératiniques humaines telles que les cheveux, contenant, dans un milieu approprié pour la teinture, au moins un précurseur de colorant d'oxydation, au moins une enzyme alcool oxydase, au moins un substrat pour ladite enzyme et au moins une silicone aminée ou oxyalkylénée.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance à des composés colorés par un processus de condensation oxydative.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

La coloration est généralement réalisée en milieu fortement alcalin, en présence de peroxyde d'hydrogène. Toutefois, l'utilisation des milieux alcalins en présence de peroxyde d'hydrogène présente pour inconvénient d'entraîner une dégradation non négligeable des fibres, ainsi qu'une décoloration des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi la demande de brevet FR 2 769 219 décrit l'utilisation d'une enzyme uricase et de son substrat l'acide urique en coloration d'oxydation pour la teinture de fibres kératiniques. La demande EP-A-0 310 675 décrit l'utilisation de précurseur de colorant d'oxydation de type benzénique en association avec des enzymes telles que la pyranose-oxydase, la glucose-oxydase. Plus récemment, la demande de brevet FR 2 833 492 décrit l'utilisation de l'enzyme alcool-oxydase en tant que seule enzyme dans une composition de coloration d'oxydation pour la teinture de fibres kératiniques.

Le but de la présente invention est de fournir de nouvelles compositions pour la teinture des fibres kératiniques par coloration d'oxydation stables, utilisant un système oxydant différent du peroxyde d'hydrogène.

De manière avantageuse et inattendue, la demanderesse vient maintenant de découvrir qu'il est possible d'atteindre ce but en utilisant au moins un précurseur de colorant d'oxydation, au moins une enzyme alcool oxydase, au moins un substrat pour ladite enzyme et au moins une silicone aminée ou oxyalkylénée dans une composition pour la teinture des fibres kératiniques, en particulier des fibres kératiniques humaines telles que les cheveux.

Les compositions selon la présente invention présentent l'avantage de conduire à l'obtention de teintures de couleurs puissantes, peu sélectives et résistantes, ces compositions sont capables d'engendrer des nuances variées de couleur intense et uniforme, sans dégradation significative du cheveu. En outre, on a noté que l'utilisation d'une telle composition améliore la tenue des cheveux permanentés et diminue la porosité du cheveu.

D'autres caractéristiques, aspects, objets et avantages de la présente invention apparaîtront encore plus clairement à la lecture de la description et des exemples qui suivent.

Rappelons que l'on entend désigner par silicone, en conformité avec l'acceptation générale, tous polymères ou oligomères organosiliciés à structure linéaire ou cyclique, ramifiée ou réticulée, de poids moléculaire variable, obtenus par polymérisation et/ou polycondensation de silanes convenablement fonctionnarisés, et constitués pour l'essentiel par une répétition de motifs principaux dans lesquels les atomes de silicium sont reliés entre eux par des atomes d'oxygène (liaison siloxane -Si-O-Si-), des radicaux hydrocarbonés éventuellement substitués, étant directement liés par l'intermédiaire d'un atome de carbone sur lesdits atomes de silicium. Les radicaux hydrocarbonés les plus courants sont les radicaux alkyles, notamment en C₁-C₁₀, et en particulier méthyle, les radicaux fluoroalkyles dont la partie alkyle est en C₁-C₁₀, les radicaux aryles et en particulier phényle.

Plus particulièrement, on désigne par silicone aminée toute silicone comportant au moins une fonction amine primaire, secondaire, tertiaire ou un groupement ammonium quaternaire.

Au sens de la présente invention, on désigne aussi par silicone polyoxyalkylénée toute silicone comportant au moins un groupement oxyalkyléné de type (-CₓH₂ₓO-)ₐ dans lequel x peut varier de 2 à 6, et a est supérieur ou égal à 2.

Les compositions selon la présente invention comprennent une silicone aminée ou oxyalkylénée ou un mélange de plusieurs silicones aminées ou de plusieurs silicones oxyalkylénées, ou encore de plusieurs silicones aminées et oxyalkylénées.

Conformément à l'invention, les silicones aminées sont choisies parmi :
(a) les composés correspondant à la formule suivante : 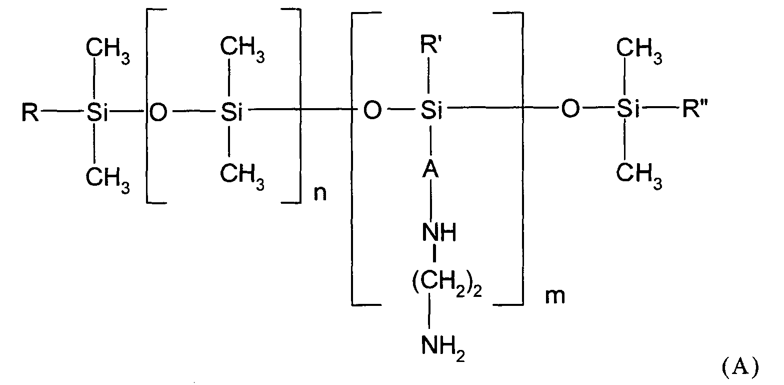 dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.
   Selon une première possibilité, R, R', R", identiques ou différents, représentent un radical alkyle en en C₁-C₄ ou hydroxyle, A représente un radical alkylène en C₃ et m et n sont tels que la masse moléculaire moyenne en poids du composé est comprise entre 5 000 et 500 000 environ. Les composés de ce type sont dénommés dans le dictionnaire CTFA, "amodiméthicone".
   Selon une deuxième possibilité, R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000.
   Dans cette catégorie de composés, on peut citer entre autres, le produit Belsil®ADM 652, commercialisée par Wacker.
   Selon une troisième possibilité, R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy / alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.
   Plus particulièrement, on peut citer le produit FluidWR® 1300, commercialisé par Wacker.
   Selon une quatrième possibilité, R, R" représentent un radical hydroxyle, R' représente un radical méthyle et A est un radical alkylène, en C₄-C₈, de préférence en C₄. Par ailleurs, m et n sont tels que la masse moléculaire moyenne en poids du composé est comprise entre 2000 et 10⁶. Plus particulièrement, n est compris entre 0 et 1999 et m est compris entre 1 et 2000, la somme de n et m étant comprise entre 1 et 2000.
   Un produit de ce type est notamment commercialisé sous la dénomination DC28299 par Dow Corning.
   Notons que la masse moléculaire de ces silicones est déterminée par chromatographie par perméation de gel (température ambiante, étalon polystyrène ; colonnes µ styragem ; éluant THF ; débit de 1 mm/m ; on injecte 200 µl d'une solution à 0,5 % en poids de silicone dans le THF et l'on effectue la détection par réfractométrie et UV-métrie).
(b) les composés répondant à la formule (B) suivante :

   (R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (B)

   dans laquelle,
   T est un atome d'hydrogène, ou un radical phényle, hydroxyle (-OH), ou alkyle en C₁-C₈, et de préférence méthyle,
   a désigne le nombre 0 ou un nombre entier de 1 à 3, et de préférence 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R1 est un radical monovalent de formule -C_{q}H_{2q}L dans laquelle q est un nombre de 2 à 8 et L est un groupement aminé éventuellement quaternisé choisi parmi les groupements :

      -N(R²)-CH₂-CH₂-N(R²)₂ ; -N(R²)₂ ; -N⁺(R²)₃ Q⁻ ;

      -N⁺(R²) (H)₂ Q⁻ ; -N⁺(R²)₂HQ⁻ ;

      -N(R²)-CH₂-CH₂-N⁺(R²)(H)₂ Q⁻,

      dans lesquels R² peut désigner un atome d'hydrogène, un phényle, un benzyle, ou un radical hydrocarboné saturé monovalent, par exemple un radical alkyle en C₁-C₂₀, et Q- représente un ion halogénure tel que par exemple fluorure, chlorure, bromure ou iodure.
      Un produit correspondant à cette définition est le polymère dénommé dans le dictionnaire CTFA "triméthylsilylamodiméthicone", répondant à la formule (C) suivante : 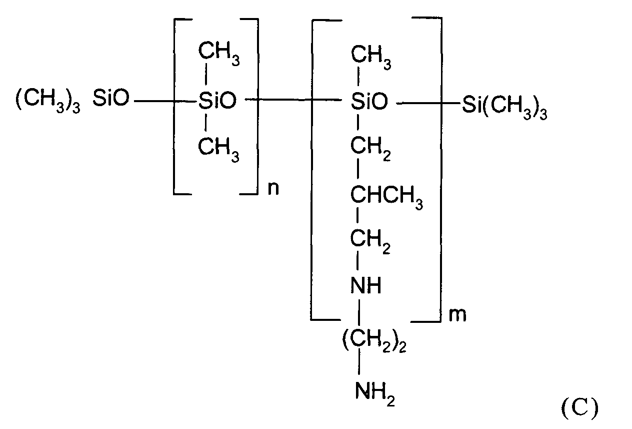 dans laquelle n et m ont les significations données ci-dessus [cf formule (B)].
      De tels composés sont décrits par exemple dans EP 95238; un composé de formule (C) est par exemple vendu sous la dénomination Q2-8220 par la société OSI.
(c) les composés répondant à la formule (D) suivante : 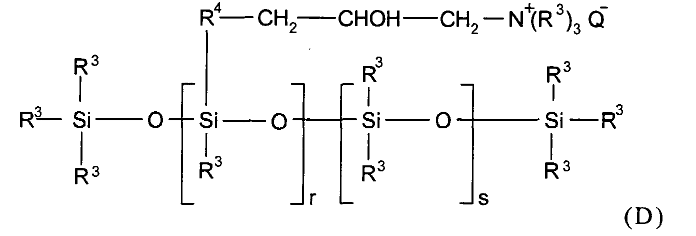
dans laquelle,
R³ représente un radical hydrocarboné monovalent en C₁-C₁₈, et en particulier un radical alkyle en C₁-C₁₈, ou alcényle en C₂-C₁₈, par exemple méthyle ;
R⁴ représente un radical hydrocarboné divalent, notamment un radical alkylène en C₁-C₁₈ ou un radical alkylèneoxy divalent en C₁-C₁₈, par exemple en C₁-C₈;
Q- est un ion halogénure, notamment chlorure ;
r représente une valeur statistique moyenne de 2 à 20 et en particulier de 2 à 8 ;
s représente une valeur statistique moyenne de 20 à 200 et en particulier de 20 à 50.

De tels composés sont décrits plus particulièrement dans le brevet US 4185087.

Un composé entrant dans cette classe est celui vendu par la Société Union Carbide sous la dénomination "Ucar Silicone ALE 56".

Lorsque ces composés sont mis en oeuvre, une forme de réalisation particulièrement intéressante est leur utilisation conjointe avec des agents de surface cationiques et/ou non ioniques.

A titre exemple, on peut utiliser le produit vendu sous la dénomination "Emulsion Cationique DC 929" par la Société Dow Corning, qui comprend, outre l'amodiméthicone, un agent de surface cationique comprenant un mélange de produits répondant à la formule : 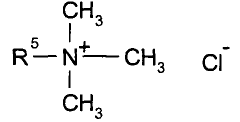 dans laquelle, R⁵ désigne des radicaux alcényle et/ou alcoyle en C₁₄-C₂₂ dérivés des acides gras du suif, et connu sous la dénomination CTFA "tallowtrimonium chloride",en association avec un agent de surface non ionique de formule :
C₉H₁₉-C₆H₄-(OC₂H₄)₁₀-OH, connu sous la dénomination CTFA "Nonoxynol 10".

On peut également utiliser par exemple le produit vendu sous la dénomination "Emulsion Cationique DC 939" par la Société Dow Corning, qui comprend, outre l'amodiméthicone, un agent de surface cationique qui est le chlorure de triméthylcétylammonium et un agent de surface non ionique de formule : C₁₃H₂₇-(OC₂H₄)₁₂-OH, connu sous la dénomination CTFA "tridéceth-12".

Un autre produit commercial utilisable selon l'invention est le produit vendu sous la dénomination "Dow Corning Q2 7224" par la Société Dow Corning, comportant en association le triméthylsilylamodiméthicone de formule (C) décrite ci-dessus, un agent de surface non ionique de formule : C₈H₁₇-C₆(H₄-(OCH₂CH₂)₄₀-OH, connu sous la dénomination CTFA "octoxynol-40", un second agent de surface non ionique de formule: C₁₂H₂₅-(OCH₂-CH₂)₆-OH, connu sous la dénomination CTFA "isolaureth-6", et du propylèneglycol.

Conformément à l'invention, les silicones oxyalkylénées sont choisies parmi les composés de formules générales (I), (II), (III), (IV) ou (V): 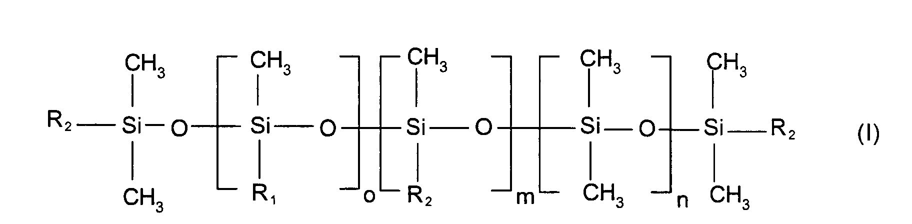 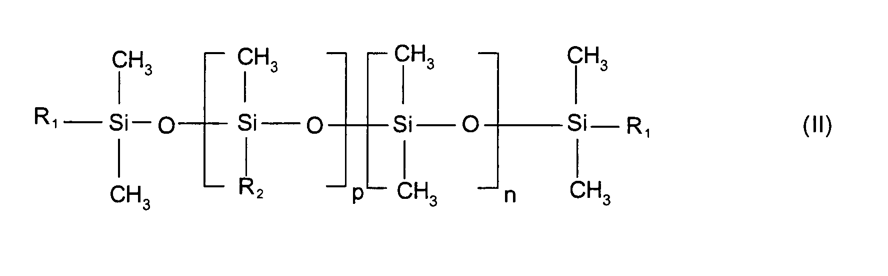 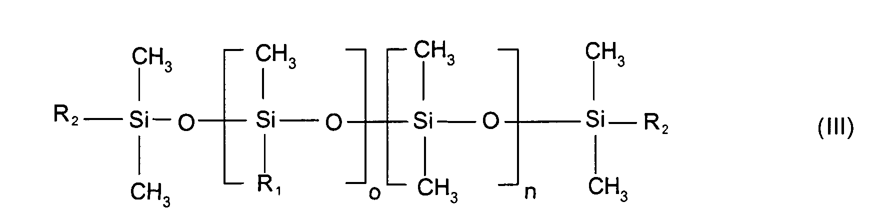 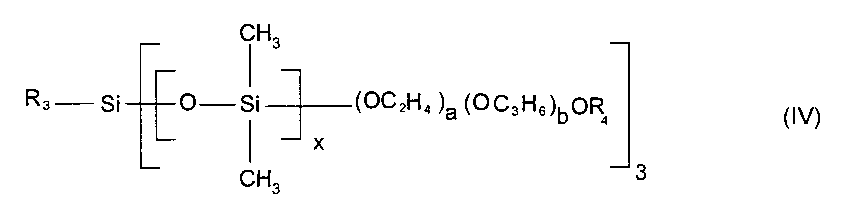 formules (I), (II), (III) et (IV) dans lesquelles:
- R₁, identique ou différent, représente un radical alkyle, linéaire ou ramifié, en C₁-C₃₀ ou phényle,
- R₂, identique ou différent, représente un radical -C_{c}H_{2c}-O-(C₂H₄O)ₐ(C₃H₆O)_{b}-R₅ ou un radical -C_{c}H_{2c}-O-(C₄H₈0)ₐ-R₅,
- R₃, R₄, identiques ou différents, désignent un radical alkyle, linéaire ou ramifié, en C₁ à C₁₂, et de préférence le radical méthyle,
- R₅, identique ou différent, est choisi parmi un atome d'hydrogène, un radical alkyle, linéaire ou ramifié, comprenant de 1 à 12 atomes de carbone, un radical alcoxy, linéaire ou ramifié, comprenant de 1 à 6 atomes de carbone, un radical acyle, linéaire ou ramifié, comprenant de 2 à 30 atomes de carbone, un radical hydroxyle, -SO₃M, aminoalcoxy en C₁-C₆ éventuellement substitué sur l'amine, aminoacyle en C₂-C₆ éventuellement substitué sur l'amine, -NHCH₂CH₂COOM, -N(CH₂CH₂COOM)₂, aminoalkyle éventuellement substitué sur l'amine et sur la chaîne alkyle, carboxyacyle en C₂-C₃₀, un groupement éventuellement substitué par un ou deux radicaux aminoalkyle substitués, -CO(CH₂)_{d}COOM, -COCHR₇(CH₂)_{d}COOM, -NHCO(CH₂)_{d}OH, -NH₃Y, un groupement phosphate,
- M, identique ou différent, désigne un atome d'hydrogène, Na, K, Li, NH₄ ou une amine organique,
- R₇ désigne un atome d'hydrogène ou un radical SO₃M,
- d varie de 1 à 10,
- m varie de 0 à 20,
- n varie de 0 à 500,
- o varie de 0 à 20,
- p varie de 1 à 50,
- a varie de 0 à 50,
- b varie de 0 à 50,
- a + b est supérieur ou égal à 2,
- c varie de 0 à 4,
- x varie de 1 à 100,
- Y représente un anion minéral ou organique monovalent tel que halogénure (chlorure, bromure), sulfate, carboxylate (acétate, lactate, citrate),
sous réserve que lorsque la silicone est de formule (II) avec R₅ désignant hydrogène alors n est supérieur à 12.

De telles silicones sont par exemple commercialisées par la société GOLDSCHMIDT sous les dénominations commerciales ABIL WE 09, ABIL EM 90, ABIL B8852, ABIL B8851, ABIL B 8843, ABIL B8842, par la société DOW CORNING sous les dénominations FLUID DC 190, DC 3225 C, Q2-5220, Q25354, Q2-5200, par la société RHONE POULENC sous les dénominations SILBIONE HUILE 70646, RHODORSIL HUILE 10634, par la société GENERAL ELECTRIC sous les dénominations SF1066, SF1188, par la société SWS SILICONES sous la dénomination SILICONE COPOLYMER F 754, par la société AMERCHOL sous la dénomination SILSOFT BEAUTY AID SL, par la société SHIN-ETSU sous la dénomination KF 351, par la société WACKER sous la dénomination BELSIL DMC 6038, par la société SILTECH sous les dénominations SILWAX WD-C, SILWAX WD-B, SILWAX WD-IS, SILWAX WSL, SILWAX DCA 100, SILTECH AMINE 65, par la société FANNING CORPORATION sous les dénominations FANCORSIL SLA, FANCORSIL LIM1, par la société PHOENIX sous la dénomination PECOSIL.

Ces silicones sont notamment décrites dans les brevets US-A-5 070 171, US-A-5149765, US-A-5093452 et US-A-5091493.

De préférence, on utilise les silicones polyoxyalkylénées répondant aux formules générales (II) ou (III). Plus particulièrement, ces formules répondent à au moins une des, et de préférence toutes les, conditions suivantes :
- c est égal à 2 ou 3.
- R₁ désigne le radical méthyle.
- R₅ représente un radical méthyle, un radical acyle en C₁₂-C₂₂, CO(CH₂)_{d}COOM.
- a varie de 2 à 25 et plus particulièrement de 2 à 15.
- b est égal à 0.
- n varie de 0 à 100.
- p varie de 1 à 20.

Les silicones polyoxyalkylénées selon l'invention peuvent également être choisies parmi les silicones de formule (V) suivante :

([ Z (R₂SiO)_{q}R'₂SiZO][( CₙH₂ₙO)ᵣ])ₛ (V)

formule (V) dans laquelle:
- R₂ et R'₂, identiques ou différents, représentent un radical hydrocarboné monovalent en C₁-C₃₀,
- n est un nombre entier allant de 2 à 4,
- q est un nombre supérieur ou égal à 4, de préférence compris entre 4 et 200 et encore plus particulièrement entre 4 et 100.
- r est un nombre supérieur ou égal à 4, de préférence compris entre 4 et 200 et encore plus particulièrement entre 5 et 100.
- s est un nombre supérieur ou égal à 4, de préférence compris entre 4 et 1 000 et encore plus particulièrement entre 5 et 300.
- Z représente un groupe organique divalent qui est lié à l'atome de silicium adjacent par une liaison carbone-silicium et au bloc polyoxyalkylène (CₙH₂ₙO) par un atome d'oxygène,
- le poids moléculaire moyen de chaque bloc siloxane est compris entre environ 400 et environ 10 000, celui de chaque bloc polyoxyalkylène étant compris entre environ 300 et environ 10 000,
- les blocs siloxane représentent de 10% environ à 95% environ en poids du copolymère bloc,
- le poids moléculaire moyen en nombre du copolymère bloc pouvant aller de 2 500 à 1 000 000 et de préférence compris entre 3 000 et 200 000 et encore plus particulièrement entre 6 000 et 100 000.

R₂ et R'₂ sont préférentiellement choisis parmi le groupe comprenant les radicaux alkyles linéaires ou ramifiés comme par exemple les radicaux méthyle, éthyle, propyle, butyle, pentyle, hexyle, octyle, décyle, dodécyle, les radicaux aryles comme par exemple phényle, naphtyle, les radicaux aralkyls ou alkylaryles comme par exemple benzyle, phényléthyle, les radicaux tolyle, xylyle.

Z est de préférence -R"-, -R"-CO-, -R"-NHCO-, -R"-NH-CO-NH-R"'-, -R"-OCONH-R"'-NHCO-, où R" est un groupe alkylène divalent, linéaire ou ramifié en C₁-C₆, comme par exemple l'éthylène, le propylène ou le butylène, linéaire ou ramifié et R"' est un groupe alkylène divaient ou un groupe arylène divalent comme -C₆H₄-, -C₆H₄-C₆H₄-, -C₆H₄-CH₂-C₆H₄-, -C₆H₄-C(CH₃)₂C₆H₄-.

Encore plus préférentiellement, Z représente un radical alkylène divalent, plus particulièrement le radical -C₃H₆- ou le radical C₄H₈, linéaires ou ramifiés.

La préparation des copolymères blocs mis en oeuvre dans le cadre de la présente invention est décrite dans la demande européenne EP 0 492 657 A1, dont l'enseignement est inclus dans la présente description.

De tels produits sont par exemple commercialisés sous la dénomination SILICONE FLUID FZ-2172 par la société OSI.

Les silicones utilisées dans les compositions selon l'invention peuvent se présenter sous forme de solutions aqueuses ou éventuellement sous forme de dispersions ou d'émulsions aqueuses.

La concentration en silicones aminées et/ou oxyalkylénées est comprise entre 0,05 et 30% et de préférence entre 0,1 et 15% en poids par rapport au poids total de la composition.

Dans le cadre de la présente invention, les enzymes alcool oxydases utilisées dans la composition tinctoriale conforme à l'invention appartiennent à la classe E.C.1.1.3 de la nomenclature des enzymes, (voir Enzyme Nomenclature, Academic Press Inc ; 1992).

Lesdites enzymes sont choisies parmi les alcool primaire oxydases ( EC1.1.3.13), les alcool secondaire oxydases (EC 1.1.3.18), les alcool à longue chaîne hydrocarbonée oxydases (EC 1.1.3.20), les alcool polyvinylique oxydases ( EC 1.1.3.30), l'alcool vanillique oxydase (EC 1.1.3.38), les alcool aromatique oxydases (EC 1.1.3.7) encore appelées aryl alcool oxydases.

De préférence l'enzyme utilisée dans la composition selon l'invention est une alcool primaire oxydase ( EC1.1.3.13).

Les enzymes alcool oxydases forment une classe particulière d'enzymes oxydo-réductase à 2 électrons.

L'enzyme alcool oxydase utilisée dans la composition tinctoriale selon l'invention peut être issue d'un extrait de végétaux, d'animaux, de microorganismes (bactérie, champignon, levure, microalgue ou virus), de cellules différenciées ou dédifférenciées, obtenues *in vivo ou in vitro,* modifiées ou non modifiées génétiquement, ou synthétiques (obtenues par synthèse chimique ou biotechnologique).

A titre d'exemples, l'enzyme alcool oxydase peut être issue d'une des espèces suivantes : *Rhodococcus erythropolis, Pseudomonas pseudoalcaligenes* qui sont des bactéries, *Aspergillus niger, Kamagataella pastoris, Phanerochaete chrysosporium, Polyporus obtusus, Hansenula polymorpha, Poria contigua, Penicillium simplicissimum, Pleurotus pulmonarius* (champignons), *Pichia sp. (pastoris, methanolica, angusta)* et *Candida sp. (boidinii, albicans*, *tropicalis)* (levures), *Pinus strobus* qui est une espèce d'origine végétale, *Gastropode mollusc, Manduca sexta* qui sont d'origine animale.

De préférence l'enzyme utilisée dans la composition selon l'invention est une alcool oxydase provenant de *Pichia pastoris.*

Généralement, la concentration en enzyme alcool oxydase utilisée dans la composition tinctoriale est comprise entre 0,05% et 20% en poids, de préférence comprise entre 0,1 et 10% en poids et de manière encore plus préférée entre 0,5 et 8% en poids par rapport au poids de cette composition

L'activité enzymatique des enzymes alcool oxydases utilisées conformément à l'invention peut être définie à partir de l'oxydation du donneur en condition aérobie. Unité U correspond à la quantité d'enzyme conduisant à la génération de 1 µmole de peroxyde d'hydrogène par minute à un pH donné et à une température de 25°C.

De façon préférentielle la quantité d'alcool oxydase est comprise entre 10³U et 10⁵ U, de préférence entre 2.10³U et 5.10⁴ U pour 100g de composition tinctoriale.

Le ou les substrats pour ladite enzyme sont encore appelés donneurs pour l'enzyme.

Le substrat pour l'enzyme est de préférence un alcool choisi parmi les alcools primaires, secondaires, les alcools à longue chaîne hydrocarbonée et les alcools aromatiques. Par exemple à titre de donneur pour les alcool primaire oxydases, on peut citer les alcools primaires contenant de 1 à 6 atomes de carbone ; à titre de donneur pour les aryl alcool oxydases : l'alcool benzylique, le 4-terbutyl benzylique alcool, le 3-hydroxy-4- méthoxybenzyl alcool, le vératryl alcool, le 4-méthoxybenzyl alcool, l'alcool cinnamique ; le 2,4 hexadiéne-1-ol peut également être utilisé en tant que donneur pour les aryl alcool oxydases.

Selon une autre variante de l'invention, le substrat pour l'enzyme est un composé portant au moins une fonction alcool aliphatique ou aromatique, approprié pour réagir avec l'enzyme utilisée. Le composé portant au moins une fonction alcool aliphatique ou aromatique peut notamment être un précurseur de colorant d'oxydation ou encore un adjuvant cosmétiquement acceptable par exemple un polymère, un tensioactif, un conservateur porteur d'au moins une fonction alcool. De façon encore plus préférée, le substrat pour l'enzyme est un précurseur de colorant d'oxydation portant au moins une fonction alcool aliphatique ou aromatique. Par exemple, la N-(β-hydroxypropyl)-paraphénylènediamine qui porte une fonction alcool primaire peut avoir la fonction de base d'oxydation et de substrat pour l'alcool oxydase. De même, les coupleurs d'oxydation, tels que le méta- ou le para-aminophénol, peuvent remplir les deux fonctions. De tels précurseurs sont décrits ci-après. Dans cette variante, l'utilisation d'autres substrats de l'enzyme est facultative.

Ainsi la présente demande vise une composition pour la teinture des fibres kératiniques, en particulier des fibres kératiniques humaines telles que les cheveux, contenant, dans un milieu approprié pour la teinture, au moins les composés suivants : un précurseur de colorant d'oxydation ; au moins une enzyme alcool oxydase ; au moins un substrat, portant une fonction alcool, pour ladite enzyme et au moins une silicone aminée ou oxyalkylénée, ledit substrat pouvant être substitué (c'est-à-dire remplacé) en tout ou en partie par le précurseur de colorant d'oxydation dans le cas où il porte au moins une fonction alcool aliphatique ou aromatique.

L'utilisation de la composition conforme à l'invention permet de diminuer les risques associés à la manipulation de peroxyde d'hydrogène. De plus, la concentration en conservateurs des compositions selon la présente invention peut être diminuée par l'apport de composés possédant une fonction alcool qui possèdent également des propriétés de conservateur.

Généralement, la concentration du ou des substrats pour l'enzyme est comprise entre 0,01% et 60% en poids par rapport au poids total de la composition et de préférence comprise entre 0,05% et 30% en poids par rapport au poids total de la composition.

Les bases d'oxydation classiques peuvent notamment être choisies parmi les paraphénylènediamines, les bis-phénylalkylènediamines, les para-aminophénols, les ortho-aminophénols et les bases hétérocycliques et leurs sels d'addition.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène, la 3-hydroxy 1-(4' aminophenyl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 2-chloro phénol, le 4-amino 3-chloro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino 2,6-dichlorophénol, le 4-amino-6-[((5'-amino-2'-hydroxy-3'-méthyl)phényl)méthyl]-2-méthylphénol, le bis(5'-amino-2'-hydroxy)phénylméthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ;
ainsi que leurs sels d'addition avec un acide..

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Généralement la concentration de la ou des bases d'oxydation est comprise entre 0,0001 et 20%, de préférence entre 0,005 à 6% en poids par rapport au poids total de la composition.

Parmi les coupleurs d'oxydation classiques, on peut notamment citer les métaphénylènediamines, les méta-aminophénols, les métadiphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement la concentration du ou des coupleurs d'oxydation est comprise entre 0,0001 et 20%, de préférence entre 0,005 à 6% en poids par rapport au poids total de la composition.

D'une manière générale, les sels d'addition avec un acide utilisables pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

Les sels d'addition avec une base utilisables dans le cadre de l'invention sont par exemple choisis parmi les sels d'addition avec la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs méthiniques, azométhiniques, triarylméthaniques, indoaminiques et les colorants directs naturels. De préférence, la composition selon l'invention comprend au moins un colorant choisi parmi les colorants directs cationiques et les colorants directs naturels.

Parmi les colorants directs cationiques utilisables selon l'invention on peut citer les colorants directs azoïques cationiques décrits dans les demandes de brevets WO 95/15144, WO-95/01772 et EP-714954.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono) méthyl]-pyridinium.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

La composition tinctoriale conforme à l'invention peut également renfermer divers adjuvants additionnels. Ces agents additionnels sont utilisés classiquement dans les compositions pour la teinture des cheveux, ce sont par exemple des agents antioxydants, des agents de pénétration, des agents séquestrants, des parfums, des tampons, des agents dispersants, des tensioactifs, des agents conditionneurs tels que par exemple des silicones additionnelles volatiles ou non volatiles, modifiées ou non modifiées, différentes des silicones aminées ou oxyalkylénées utilisées dans les compositions selon l'invention ; des polymères cationiques, des cations, des agents filmogènes, des céramides, des agents conservateurs, des agents opacifiants, des vitamines ou provitamines.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. Ce solvant peut être le cas échéant un substrat de l'enzyme comme l'éthanol, l'isopropanol. Cela peut être également un composé non substrat de l'enzyme tel que les éthers de polyols comme le 2-butoxyéthanol, le propylèneglycol, le monométhyléther de propylèneglycol, le monoéthyléther et le monométhyléther du diéthylèneglycol, le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 6 et 11 environ, et de préférence entre 7 et 10 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 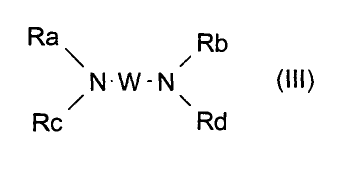 dans laquelle W est un reste propylène éventuellement substitué par un groupement hydroxyle ou un radical alkyle en C₁-C₄ ; Ra, Rb, Rc et Rd, identiques ou différents, représentent un atome d'hydrogène, un radical alkyle en C₁-C₄ ou hydroxyalkyle en C₁-C₄.

La composition tinctoriale peut se présenter sous des formes diverses, telles que sous forme de liquides, de crèmes, de gels, ou sous toute autre forme appropriée pour réaliser une teinture des fibres kératiniques, et notamment des cheveux humains.

Lorsque les colorants d'oxydation et la ou les alcool oxydases sont présents au sein de la même composition prête à l'emploi, ladite composition est de préférence exempte d'oxygène gazeux de manière à éviter toute oxydation prématurée du ou des colorants d'oxydation.

L'invention a également pour objet un procédé de teinture des fibres kératiniques, et en particulier des fibres kératiniques humaines telles que les cheveux, tel qu'on applique sur ces fibres au moins une composition pour la teinture selon l'invention, la durée de cette application étant la durée suffisante pour développer la coloration désirée.

La couleur est alors révélée par la mise en présence de l'enzyme alcool oxydase et de son substrat en présence d'oxygène.

La composition est appliquée sur les fibres kératiniques. Après un temps de pose de 3 à 60 minutes environ, de préférence 5 à 40 minutes environ, les fibres kératiniques sont rincées, lavées au shampooing, rincées à nouveau puis séchées.

Lorsque la composition pour la teinture est une composition sous une forme prête à l'emploi, elle comprend, dans un milieu approprié pour la teinture des fibres kératiniques, au moins un précurseur de colorant d'oxydation, au moins une enzyme alcool oxydase, au moins un substrat pour ladite enzyme et au moins une silicone aminée ou oxyalkylénée, l'ensemble est alors stocké sous forme anaérobie, exempte d'oxygène gazeux.

Selon une variante, ce procédé comporte une étape préliminaire consistant à stocker sous forme séparée, d'une part une composition (A) comprenant, dans un milieu approprié pour la teinture des fibres kératiniques, au moins un précurseur de colorant d'oxydation et d'autre part, une composition (B) renfermant, dans un milieu approprié pour la teinture des fibres kératiniques, au moins une enzyme alcool oxydase, la composition (A) et/ou la composition (B) contenant au moins un substrat pour ladite enzyme, et la composition (A) et/ou la composition (B) contenant au moins une silicone aminée ou oxyalkylénée ; puis à procéder au mélange des compositions (A) et (B) au moment de l'emploi avant d'appliquer ce mélange sur les fibres kératiniques.

Selon une variante de l'invention, le procédé comporte une étape préliminaire consistant à stocker sous forme séparée, d'une part une composition (A) comprenant, dans un milieu approprié pour la teinture des fibres kératiniques, au moins un précurseur de colorant d'oxydation, au moins un substrat pour ladite enzyme et au moins une silicone aminée ou oxyalkylénée et d'autre part, une composition (B) renfermant, dans un milieu approprié pour la teinture des fibres kératiniques, au moins une enzyme alcool oxydase, puis à procéder au mélange des compositions (A) et (B) au moment de l'emploi avant d'appliquer ce mélange sur les fibres kératiniques.

La couleur peut être révélée à pH acide, neutre ou alcalin. Dans le cas où le procédé est mis en oeuvre au moyen d'une composition (A) comprenant au moins un précurseur de colorant d'oxydation, au moins un substrat pour ladite enzyme et au moins une silicone aminée ou oxyalkylénée et d'une composition (B) renfermant au moins une enzyme alcool oxydase, l'enzyme peut être ajoutée à la composition de l'invention juste au moment de l'emploi ou peut être mise en oeuvre à partir d'une composition la contenant, appliquée simultanément ou séquentiellement à la composition de l'invention.

Cette composition B (dite composition oxydante) peut également renfermer divers adjuvants utilisés classiquement dans les compositions pour la teinture des cheveux et tels que définis précédemment.

Le pH de la composition B dite oxydante est tel qu'après mélange avec la composition tinctoriale A, le pH de la composition résultante appliquée sur les fibres kératiniques varie de préférence entre 6 et 11 environ, et encore plus préférentiellement entre 7 et 10. Il peut être ajusté à la valeur désirée au moyen d'agents acidifiants ou alcalinisants habituellement utilisés en teinture des fibres kératiniques et tels que définis précédemment.

De préférence, l'application de la composition selon l'invention est effectuée à une température comprise entre la température ambiante et 220°C, de préférence entre la température ambiante et 60°C.

Un autre objet de l'invention est un dispositif à plusieurs compartiments ou "kit" de teinture dans lequel un premier compartiment renferme la composition (A) telle que définie ci-dessus et un deuxième compartiment renferme la composition (B) telle que définie ci-dessus. Ce dispositif peut être équipé d'un moyen permettant de délivrer sur les cheveux le mélange souhaité, tel que les dispositifs décrits dans le brevet FR-2 586 913 au nom de la demanderesse.

Les exemples qui suivent servent à illustrer l'invention sans toutefois présenter un caractère limitatif.

### EXEMPLES

On prépare les compositions suivantes

| Constituant | Comp.1 | Comp.2 | Comp.3 | Comp.4 | Comp.5 |
|---|---|---|---|---|---|
| SILWAX WD IS | 0,3 g | | | | |
| SILPHOS A 100 | | 0,5 g | | | |
| SILWET L77 | | | 0,4 g | | |
| Dow Corning 939 emulsion | | | | 1,2 g | |
| Dow Corning Q2-8220 fluid | | | | | 1 g |
| Ethanol | 25 g | 25 g | 25 g | 25 g | 25 g |
| Paraphénylène diamine | 3.10⁻³ mole | 3.10⁻³ mole | 3.10⁻³ mole | 3.10⁻³ mole | 3.10⁻³ mole |
| Métaamino phénol | 3.10⁻³ mole | 3.10⁻³ mole | 3.10⁻³ mole | 3.10⁻³ mole | 3.10⁻³ mole |
| Alcool oxydase | 20000 unités | 20000 unités | 20000 unités | 20000 unités | 20000 unités |
| 2-amino-2-méthyl-1-propanol qs | pH 7 | pH 7 | pH 7 | pH 7 | pH 7 |
| Eau distillée qsp | 100 g | 100 g | 100 g | 100 g | 100 g |

Dow Corning 939 emulsion : Polydiméthylsiloxane à groupements aminoéthylaminopropyle à 35% dans l'eau. Dow Corning Q2-8220 fluid : mélange de polydiméthylsiloxane à groupements aminoéthylaminoisobutyle/polydiméthylsiloxane

L'alcool oxydase utilisée est celle commercialisée par la société Biozyme Laboratories sous forme liquide à la concentration de 1980 unités/ml.

L'unité U correspond à la quantité d'enzyme conduisant à la génération de 1 µmole de peroxyde d'hydrogène par minute à pH 7,5 (tampon phosphate 100mM) et à une température de 25°C.

On applique les compositions ci-dessus sur des mèches de cheveux gris naturels et permanentés à 90% de blancs et on laisse poser pendant 30 minutes. Le rapport de bain est fixé à 5. L'alcool oxydase est ajoutée extemporanément. Les cheveux ont ensuite été rincés, lavés avec un shampooing standard, puis séchés.

Les cheveux ont été teints dans des nuances vert kaki.

## Revendications

1. Composition pour la teinture des fibres kératiniques, en particulier des fibres kératiniques humaines telles que les cheveux, contenant, dans un milieu approprié pour la teinture, au moins les composés suivants : un précurseur de colorant d'oxydation ; au moins une enzyme alcool oxydase ; au moins un substrat, portant une fonction alcool, pour ladite enzyme et au moins et au moins une silicone aminée ou oxyalkylénée, ledit substrat pouvant être substitué en tout ou en partie par le précurseur de colorant d'oxydation dans le cas où ledit précurseur porte au moins une fonction alcool aliphatique ou aromatique.

2. Composition selon la revendication 1 telle que la silicone est une silicone aminée correspondant à la formule (A) suivante : 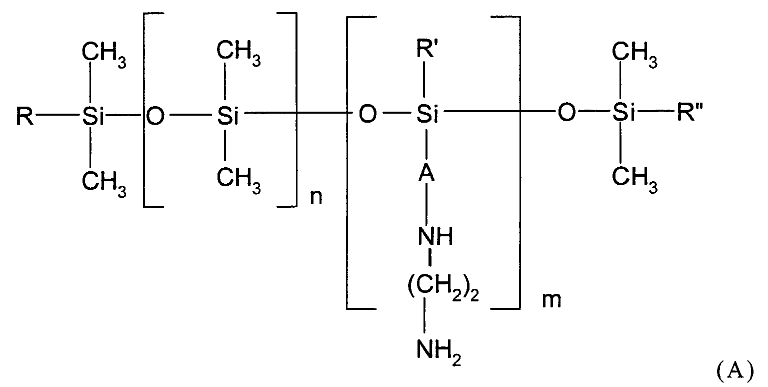 dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.

3. Composition selon la revendication 1 telle que la silicone est une silicone aminée correspondant à la formule (B) suivante :
(R¹)ₐ(T)₃₋ₐ-Si[OSi(T)₂]ₙ-[OSi(T)_{b}(R¹)_{2-b}]ₘ-OSi(T)₃₋ₐ-(R¹)ₐ
dans laquelle : T est un atome d'hydrogène, ou un radical phényle, hydroxyle, ou alkyle en C₁-C₈, et de préférence méthyle,
a désigne le nombre 0 ou un nombre entier de 1 à 3, et de préférence 0,
b désigne 0 ou 1, et en particulier 1,
m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
R¹ est un radical monovalent de formule -C_{q}H_{2q}L dans laquelle q est un nombre de 2 à 8 et L est un groupement aminé éventuellement quaternisé choisi parmi les groupements :
-N(R²)-CH₂-CH₂-N(R²)₂ ; -N(R²)₂ ; -N⁺(R²)₃ Q⁻ ;
-N⁺(R²) (H)₂ Q⁻ ; -N⁺(R²)₂HQ⁻ ;
-N(R²)-CH₂-CH₂-N⁺(R²)(H)₂ Q⁻,
dans lesquels R² peut désigner un atome d'hydrogène, un phényle, un benzyle, ou un radical hydrocarboné saturé monovalent, par exemple un radical alkyle en C₁-C₂₀, et Q- représente un ion halogénure tel que par exemple fluorure, chlorure, bromure ou iodure.

4. Composition selon la revendication 3 telle que la silicone aminée est une
"triméthylsilylamodiméthicone", répondant à la formule (C) suivante : 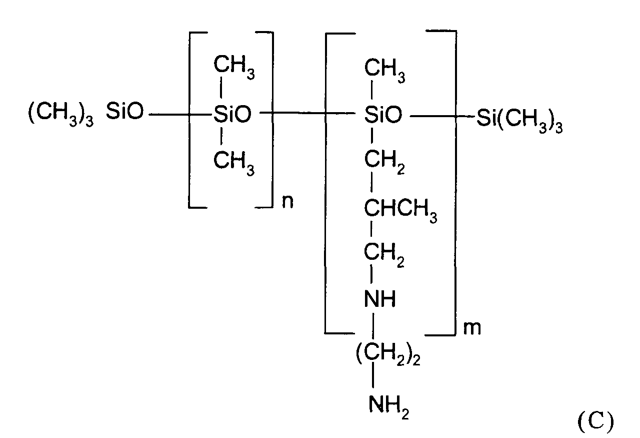 dans laquelle n et m ont les significations indiquées à la revendication 3.

5. Composition selon la revendication 1 telle que la silicone est une silicone aminée correspondant à la formule D : 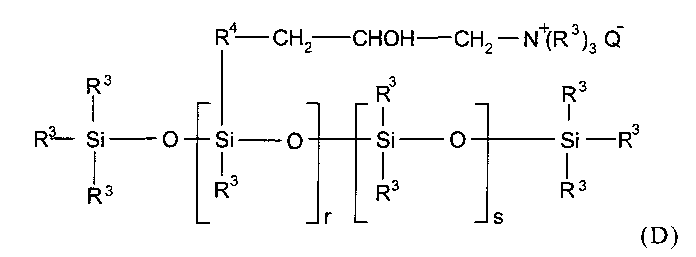 dans laquelle,
R³ représente un radical hydrocarboné monovalent en C₁-C₁₈, et en particulier un radical alkyle en C₁-C₁₈, ou alcényle en C₂-C₁₈, par exemple méthyle ;
R⁴ représente un radical hydrocarboné divalent, notamment un radical alkylène en C₁-C₁₈ ou un radical alkylèneoxy divalent en C₁-C₁₈, par exemple en C₁-C₈;
Q⁻ est un ion halogénure, notamment chlorure ;
r représente une valeur statistique moyenne de 2 à 20 et en particulier de 2 à 8 ;
s représente une valeur statistique moyenne de 20 à 200 et en particulier de 20 à 50.

6. Composition selon la revendication 1 telle que la silicone est une silicone oxyalkylénée choisie parmi les composés de formules générales (I), (II), (III), (IV) ou (V) : 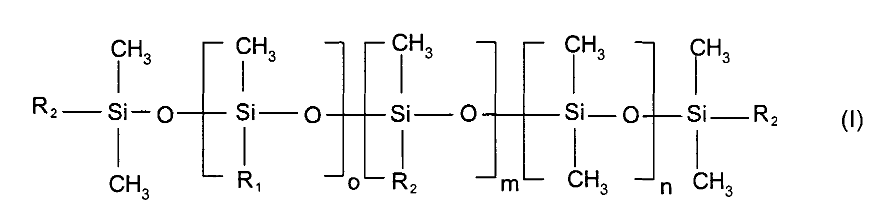 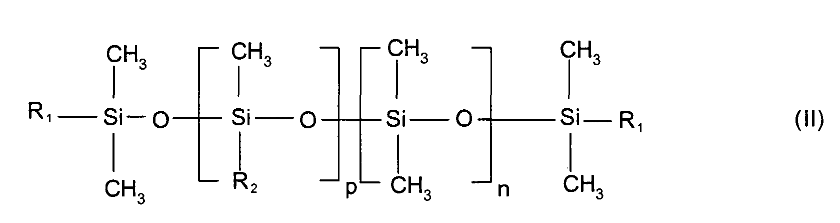 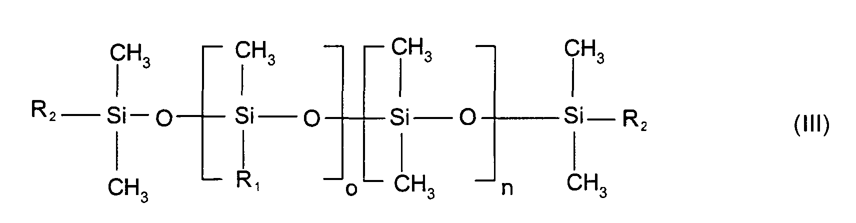 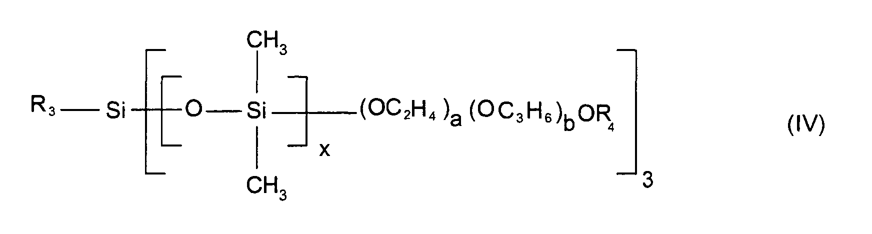 dans lesquelles:
- R₁, identique ou différent, représente un radical alkyle, linéaire ou ramifié, en C₁-C₃₀ ou phényle,
- R₂, identique ou différent, représente un radical -C_{c}H_{2c}-O-(C₂H₄O)ₐ(C₃H₆O)_{b}-R₅ ou un radical -C_{c}H_{2c}-O-(C₄H₈O)ₐ-R₅,
- R₃, R₄, identiques ou différents, désignent un radical alkyle, linéaire ou ramifié, en C_{1 à} C₁₂, et de préférence le radical méthyle,
- R₅, identique ou différent, est choisi parmi un atome d'hydrogène, un radical alkyle, linéaire ou ramifié, comprenant de 1 à 12 atomes de carbone, un radical alcoxy, linéaire ou ramifié, comprenant de 1 à 6 atomes de carbone, un radical acyle, linéaire ou ramifié, comprenant de 2 à 30 atomes de carbone, un radical hydroxyle, -SO₃M, aminoalcoxy en C₁-C₆ éventuellement substitué sur l'amine, aminoacyle en C₂-C₆ éventuellement substitué sur l'amine, -NHCH₂CH₂COOM, -N(CH₂CH₂COOM)₂, aminoalkyle éventuellement substitué sur l'amine et sur la chaîne alkyle, carboxyacyle en C₂-C₃₀, un groupement éventuellement substitué par un ou deux radicaux aminoalkyle substitués, -CO(CH₂)_{d}COOM, -COCHR₇(CH₂)_{d}COOM, -NHCO(CH₂)_{d}OH, -NH₃Y, un groupement phosphate,
- M, identique ou différent, désigne un atome d'hydrogène, Na, K, Li, NH₄ ou une amine organique,
- R₇ désigne un atome d'hydrogène ou un radical SO₃M,
- d varie de 1 à 10,
- m varie de 0 à 20,
- n varie de 0 à 500,
- o varie de 0 à 20,
- p varie de 1 à 50,
- a varie de 0 à 50,
- b varie de 0 à 50,
- a + b est supérieur ou égal à 2,
- c varie de 0 à 4,
- x varie de 1 à 100,
- Y représente un anion minéral ou organique monovalent tel que halogénure (chlorure, bromure), sulfate, carboxylate (acétate, lactate, citrate),
sous réserve que lorsque la silicone est de formule (II) avec R₅ désignant hydrogène alors n est supérieur à 12,
([ Z (R₂SiO)_{q} R'₂SiZO][( CₙH₂ₙO)ᵣ])ₛ (V)
formule (V) dans laquelle:
- R₂ et R'₂, identiques ou différents, représentent un radical hydrocarboné monovalent en C₁-C₃₀,
- n est un nombre entier allant de 2 à 4,
- q est un nombre supérieur ou égal à 4, de préférence compris entre 4 et 200 et encore plus particulièrement entre 4 et 100.
- r est un nombre supérieur ou égal à 4, de préférence compris entre 4 et 200 et encore plus particulièrement entre 5 et 100.
- s est un nombre supérieur ou égal à 4, de préférence compris entre 4 et 1 000 et encore plus particulièrement entre 5 et 300.
- Z représente un groupe organique divalent qui est lié à l'atome de silicium adjacent par une liaison carbone-silicium et au bloc polyoxyalkylène (CₙH₂ₙO) par un atome d'oxygène,
- le poids moléculaire moyen de chaque bloc siloxane est compris entre environ 400 et environ 10 000, celui de chaque bloc polyoxyalkylène étant compris entre environ 300 et environ 10 000,
- les blocs siloxane représentent de 10% environ à 95% environ en poids du copolymère bloc,
- le poids moléculaire moyen en nombre du copolymère bloc pouvant aller de 2 500 à 1 000 000.

7. Composition selon l'une des revendications précédentes telle qu'elle comprend un mélange de plusieurs silicones aminées ou de plusieurs silicones oxyalkylénées, ou encore de plusieurs silicones aminées et oxyalkylénées.

8. Composition selon l'une des revendications précédentes telle la concentration en silicones aminées et/ou oxyalkylénées est comprise entre 0,05 et 30% et de préférence entre 0,1 et 15% en poids par rapport au poids total de la composition.

9. Composition selon l'une des revendications précédentes telle que l'enzyme alcool oxydase est choisie parmi les alcool primaire oxydases (EC1.1.3.13), les alcool secondaire oxydases (EC 1.1.3.18), les alcool à longue chaîne hydrocarbonée oxydases (EC 1.1.3.20), les alcool polyvinylique oxydases (EC 1.1.3.30), l'alcool vanillique oxydase (EC 1.1.3.38), les alcool aromatique oxydases (EC 1.1.3.7).

10. Composition selon la revendication 9 telle l'enzyme alcool oxydase est issue d'une des espèces suivantes : *Rhodococcus erythropolis, Pseudomonas pseudoalcaligenes, Aspergillus niger, Kamagataella pastoris, Phanerochaete chrysosporium, Polyporus obtusus, Hansenula polymorpha, Poria contigua, Penicillium simplicissimum, Pleurotus pulmoncirius, Pichia sp. (pastoris, methanolica, angusta)* et *Candida sp. (boidinii, albicans, tropicalis), Pinus strobus, Gastropode mollusc, Manduca sexta* ,de préférence *Pichia pastoris*.

11. Composition selon l'une des revendications 9 ou 10, telle que la concentration en enzyme alcool oxydase est comprise entre 0,05 et 20%, de préférence entre 0,1 et 10% et de manière encore plus préférée entre 0,5 et 8% en poids par rapport au poids total de ladite composition.

12. Composition selon l'une des revendications précédentes telle que la quantité d'alcool oxydase est comprise entre 10³U et 10⁵U, de préférence entre 2.10³U et 5.10⁴U pour 100g de composition tinctoriale.

13. Composition selon l'une des revendications précédentes telle que le substrat pour l'enzyme est un alcool choisi parmi les alcools primaires, secondaires, les alcools à longue chaîne hydrocarbonée et les alcools aromatiques.

14. Composition selon la revendication 12 telle que la concentration du ou des substrats pour l'enzyme est comprise entre 0,01% et 60% en poids par rapport au poids total de la composition et de préférence comprise entre 0,05% et 30% en poids par rapport au poids total de la composition.

15. Composition selon l'une des revendications précédentes telle qu'elle comprend en tant que précurseur de colorant d'oxydation une base d'oxydation choisie parmi les paraphénylènediamines, les bis-phénylalkylènediamines, les para-aminophénols, les ortho-aminophénols, les bases hétérocycliques et leurs sels d'addition.

16. Composition selon la revendication 14 telle que la concentration de la ou des bases d'oxydation est comprise entre 0,0001 et 20% en poids par rapport au poids total de la composition.

17. Composition selon l'une des revendications précédentes telle que la composition comprend en tant que précurseur de colorant d'oxydation un coupleur d'oxydation choisi parmi les métaphénylènediamines, les métaaminophénols, les métadiphénols, les coupleurs naphtaléniques, les coupleurs hétérocycliques ainsi que leurs sels d'addition.

18. Composition selon la revendication 16 telle que la concentration du ou des coupleurs est comprise entre 0,0001 et 20 % en poids par rapport au poids total de la composition.

19. Composition selon l'une des revendications précédentes **caractérisée par le fait qu'**elle renferme un ou plusieurs colorants directs naturels ou cationiques.

20. Procédé de teinture des fibres kératiniques et en particulier des fibres kératiniques humaines telles que les cheveux, **caractérisée par le fait qu'**on applique sur lesdites fibres au moins une composition selon l'une des revendications 1 à 19, pendant une durée suffisante pour développer la coloration désirée.

21. Procédé selon la revendication 20, **caractérisé par le fait que** la composition est une composition prête à l'emploi comprenant une composition selon l'une des revendications 1 à 19, l'ensemble étant stocké sous forme anaérobie, exempte d'oxygène gazeux.

22. Procédé selon la revendication 21, **caractérisée par le fait qu'**il comporte une étape préliminaire consistant à stocker sous forme séparée, d'une part une composition (A) comprenant, dans un milieu approprié pour la teinture, au moins un précurseur de colorant d'oxydation et d'autre part, une composition (B) renfermant, dans un milieu approprié pour les fibres kératiniques, au moins une enzyme alcool oxydase, la composition (A) et/ou la composition (B) contenant au moins un substrat pour ladite enzyme et la composition (A) et/ou la composition (B) contenant au moins une silicone aminée ou oxyalkylénée , puis à procéder au mélange des compositions (A) et (B) au moment de l'emploi avant d'appliquer ce mélange sur les fibres kératiniques.

23. Procédé selon la revendication 22, **caractérisée par le fait qu'**il comporte une étape préliminaire consistant à stocker sous forme séparée, d'une part une composition (A) comprenant, dans un milieu approprié pour la teinture, au moins un précurseur de colorant d'oxydation, au moins un substrat pour ladite enzyme et au moins une silicone aminée ou oxyalkylénée et d'autre part, une composition (B) renfermant, dans un milieu approprié pour les fibres kératiniques, au moins une enzyme alcool oxydase, puis à procéder au mélange des compositions (A) et (B) au moment de l'emploi avant d'appliquer ce mélange sur les fibres kératiniques.

24. Dispositif à plusieurs compartiments ou « Kit » de teinture, **caractérisé par le fait qu'**il comporte un premier compartiment renfermant la composition (A) telle que définie à l'une des revendications 22 ou 23 et un second compartiment renfermant la composition (B) telle que définie à l'une des revendications 22 ou 23.
